# EUROPEAN PATENT APPLICATION

(11) **EP 4 563 190 A1**
(43) Date of publication of application: **04.06.2025**
(21) Application number: 23845173.6
(22) Date of filing: 27.06.2023
(51) Int. Cl.: A61N 1/36, A61B 5/16

(54) **PULSE GENERATOR, MEDICAL SYSTEM, AND COMPUTER-READABLE STORAGE MEDIUM**

(30) Priority: 25.07.2022 CN 202210880324
(71) Applicant: Sceneray Co., Ltd., Suzhou, Jiangsu 215123 (CN)
(72) Inventor: ZHU, Haifeng, Suzhou, Jiangsu 215123 (CN); TANG, Jiandong, Suzhou, Jiangsu 215123 (CN); ZHOU, Guoxin, Suzhou, Jiangsu 215123 (CN)
(74) Representative: Cabinet Beaumont
(86) International application number: PCT/CN2023/102709
(87) International publication number: WO 2024/021960

(57) **Abstract**

Provided are a pulse generator, a medical system, and a computer-readable storage medium. The pulse generator (11) includes a processor. The processor is configured to: (S1) sense the electrophysiological activity of a patient by using an electrode wire (12) to obtain an electrophysiological signal of the patient; (S2) input the electrophysiological signal of the patient into a state classification model to obtain a state classification result of the patient; (S3) acquire desired configuration information of the pulse generator (11) corresponding to the state classification result of the patient; (S4) detect whether the current configuration information of the pulse generator (11) matches the desired configuration information, if not, execute S5, and if yes, execute S1 after a first preset duration; and (S5) update one or more stimulation parameters of the pulse generator (11) by using the desired configuration information of the pulse generator (11) and execute S1 after a second preset duration, where the second preset duration is less than the first preset duration.

## Description

This application claims priority to Chinese Patent Application No. 202210880324.3 filed with the China National Intellectual Property Administration (CNIPA) on Jul. 25, 2022, the disclosure of which is incorporated herein by reference in its entirety.

### TECHNICAL FIELD

The present application relates to the technical fields of implantable devices, deep brain stimulation, and deep learning, for example, a pulse generator, a medical system, and a computer-readable storage medium.

### BACKGROUND

With the development of science and technology and social progress, the patients are eager to improve quality of life through various treatment manners, among which medical devices, especially implantable devices, have a very broad application prospect. The implantable devices refer to medical devices that enter human bodies or cavities (mouths) in whole or in part by use of surgeries, or are configured to replace human epithelial surfaces or eye surfaces, and are left in the human bodies for 30 days (inclusive) or more or absorbed by the human bodies after surgery processes end. Stimulators are a type of the implantable devices. A stimulator, which generally includes an implantable pulse generator (IPG), an extension wire, and an electrode wire, can provide a patient with a refined electrical stimulation therapy with controllable parameters and is popular with many consumers in the market.

During the postoperative program control process, a doctor may use a program control device to adjust stimulation parameters of the stimulator and decide whether to perform program control and determine the adjustment range of the program-control parameters based on subjective feelings of the patient. In other words, program control adjustment mainly depends on subjective experience of the doctor. This leads to a significant extension of the number of times and duration of postoperative program control for the patient, which invisibly increases the economic burden of the patient and the workload of a program control physician.

CN113244533A discloses a parameter adjustment method for automatically adjusting parameters of a stimulator implanted into a patient. The method includes: receiving a marking operation using a program control device disposed in the patient in vitro, and in response to the marking operation, collecting an electroencephalogram signal of the patient using the stimulator, where the marking operation is configured to mark a state type of the patient; associating the electroencephalogram signal of the patient and the state type of the patient and storing them in a first data set so that the first data set can include multiple electroencephalogram signals of the patient and a state type corresponding to each electroencephalogram signal; training a first deep learning model using the first data set to obtain a state classification model; collecting a real-time electroencephalogram signal of the patient using the stimulator; inputting the real-time electroencephalogram signal into the state classification model to obtain a real-time state type corresponding to the real-time electroencephalogram signal; when it is detected that the real-time state type changes relative to the previous moment, acquiring parameter configuration information corresponding to the real-time state type; and adjusting the parameters of the stimulator by using the program control device according to the parameter configuration information corresponding to the real-time state type so that the stimulator can apply corresponding electrical stimulation to the patient. The method is configured to adjust the parameter configuration information according to the change in the real-time state type, without considering time intervals between state diagnoses, and frequent state diagnoses may cause rapid power consumption. SUMMARY

The present application provides a pulse generator, an apparatus, a system, and a computer-readable storage medium so that a stimulation parameter can be adaptively adjusted based on a real-time state of a patient, and two interval durations can be used for executing the next sensing step in a targeted manner.

In a first aspect, the present application provides a pulse generator. The pulse generator is implanted into a patient and includes a processor, where the processor is configured to perform the following operations.

An electrophysiological activity of the patient is sensed by using an electrode wire to obtain an electrophysiological signal of the patient.

The electrophysiological signal of the patient is input into a state classification model to obtain a state classification result of the patient.

Desired configuration information of the pulse generator corresponding to the state classification result of the patient is acquired.

Whether current configuration information of the pulse generator matches the desired configuration information is detected.

In response to the current configuration information of the pulse generator not matching the desired configuration information, at least one stimulation parameter of the pulse generator is updated by using the desired configuration information of the pulse generator, and after a second preset duration, the processor returns to perform the operation of sensing the electrophysiological activity of the patient by using the electrode wire to obtain the electrophysiological signal of the patient.

In response to the current configuration information of the pulse generator matching the desired configuration information, after a first preset duration, the processor returns to perform the operation of sensing the electrophysiological activity of the patient by using the electrode wire to obtain the electrophysiological signal of the patient.

The second preset duration is less than the first preset duration.

In a second aspect, the present application provides a medical system. The medical system includes the preceding pulse generator, the electrode wire, and a program control device.

The electrode wire is configured to sense the electrophysiological activity of the patient and deliver electrical stimulation.

The program control device is configured to establish a program control connection with the pulse generator, receive a range configuration operation, and determine an adjustable numerical range corresponding to each stimulation parameter of the at least one stimulation parameter of the pulse generator in response to the range configuration operation.

The pulse generator is configured to perform adaptive adjustment within the adjustable numerical range corresponding to each stimulation parameter.

In a third aspect, the present application provides a computer-readable storage medium storing a computer program which, when executed by a processor, causes the processor to fulfill the functions of the preceding pulse generator.

### BRIEF DESCRIPTION OF DRAWINGS

FIG. 1 is a block diagram of a medical system according to an embodiment of the present application.
FIG. 2 is a flowchart of an adaptive stimulation method according to an embodiment of the present application.
FIG. 3 is a flowchart of acquiring desired configuration information according to an embodiment of the present application.
FIG. 4 is a flowchart of acquiring a first preset duration according to an embodiment of the present application.
FIG. 5 is a block diagram of a pulse generator according to an embodiment of the present application.
FIG. 6 is a diagram illustrating the structure of a program product according to an embodiment of the present application.

### DETAILED DESCRIPTION

Technical solutions in the present application are described below in conjunction with the drawings and specific embodiments of the present application.

In the embodiments of the present application, "at least one" refers to one or more, and "multiple" refers to two or more. "And/or" describes an association relation between associated objects and indicates three relations. For example, "A and/or B" may indicate the presence of A alone, the presence of both A and B, and the presence of B alone, where A and B may be singular or plural. The character "/" generally indicates an "or" relation between the associated objects. "At least one of the following" or a similar expression thereof refers to any combination of items, including any combination of singular items or plural items. For example, at least one of a, b, or c may indicate a, b, c, "a and b", "a and c", "b and c" or "a and b and c", where a, b, and c may be singular or plural. "At least one item" may also be interpreted as "one or more items".

In the embodiments of the present application, words such as "exemplary" or "for example" are used for indicating examples, illustrations, or descriptions. Any embodiment or design solution that is described as "exemplary" or "for example" in the embodiments of the present application should not be construed as being more preferred or advantageous than other embodiments or design solutions. Specifically, the use of words such as "exemplary" or "for example" is intended to present relevant concepts in a specific manner.

One of the application fields (that is, the implantable devices) in the embodiments of the present application is briefly described below.

An implantable neurostimulation system (an implantable medical system) mainly includes a stimulator implanted into a patient and a program control device disposed in the patient in vitro. In neuromodulation technology, an electrode is mainly implanted into a particular structure (that is, a target site) in vivo through stereotactic surgery, and an electrical pulse is sent by the stimulator implanted in the patient to the target site via the electrode to modulate electrical activities and functions of a corresponding neural structure and network, thereby improving a symptom and alleviating the disease pain. The stimulator may be any one of an implantable nerve electrical stimulation apparatus, an implantable cardiac electrical stimulation system (also referred to as a cardiac pacemaker), an implantable drug delivery system (IDDS), or a wire switching apparatus. The implantable nerve electrical stimulation apparatus is, for example, a deep brain stimulation (DBS) system, an implantable cortical nerve stimulation (CNS) system, an implantable spinal cord stimulation (SCS) system, an implantable sacral nerve stimulation (SNS) system, or an implantable vagus nerve stimulation (VNS) system.

The stimulator may include an IPG, an extension wire, and an electrode wire. Disposed in the patient in vivo, the IPG provides controllable electrical stimulation energy to tissue in vivo depending on a sealed battery and circuit and delivers one or two streams of controllable particular electrical stimulation to a particular region of the tissue in vivo via the implanted extension wire and electrode wire. By being used together with the IPG, the extension wire as a transmission medium of an electrical stimulation signal transmits the electrical stimulation signal generated by the IPG to the electrode wire. The electrode wire delivers electrical stimulation to the particular region of the tissue in vivo via multiple electrode contacts. One or more electrode wires are disposed on one side or two sides of the stimulator. Multiple electrode contacts are disposed on the electrode wire and may be evenly or non-evenly arranged in the circumferential direction of the electrode wire. As an example, the electrode contacts may be arranged in the circumferential direction of the electrode wire in an array of four rows and three columns (12 electrode contacts in total). The electrode contacts may include stimulation electrode contacts and/or collection electrode contacts. An electrode contact may be, for example, in the shape of a sheet, a ring, or a dot.

In some embodiments, the stimulated tissue in vivo may be brain tissue of the patient, and the stimulated part may be a particular part of the brain tissue. When disease types of patients are different, stimulated parts are generally different, and the number of used stimulation contacts (single source or multi-source), the application of one or more (single channel or multi-channel) particular electrical stimulation signals and stimulation parameter data are also different. The applicable disease types are not limited in the embodiments of the present application and may be disease types applicable to DBS, SCS, pelvic stimulation, gastric stimulation, periphery nerve stimulation, and functional electrical stimulation. Disease types that may be treated or managed by the DBS include, but are not limited to, seizure disorders (such as epilepsy), pain, migraine, mental illnesses (such as major depressive disorder (MDD)), bipolar disorder, anxiety disorder, post-traumatic stress disorder, dysthymia, obsessive-compulsive disorder (OCD), behavior disorder, mood disorder, memory disorder, mental state disorder, movement disorders (such as essential tremor or Parkinson's disease), Huntington's disease, Alzheimer's disease, drug addiction, autism or other neurological or psychiatric disorders and impairment.

In the embodiments of the present application, when a program control connection is established between the program control device and the stimulator, the program control device may be used for adjusting stimulation parameters of the stimulator (different stimulation parameters correspond to different electrical stimulation signals), or the stimulator may sense the electrophysiological activity of a deep brain of the patient to collect an electrophysiological signal, and the adjustment of the stimulation parameters of the electrical stimulation signals of the stimulator may be continued by the collected electrophysiological signal.

The program control device may be a doctor program controller (that is, a program control device used by a doctor) or a patient program controller (that is, a program control device used by a patient). The program control device may be, for example, a tablet computer, a notebook computer, a laptop computer, a mobile phone, or another smart terminal device.

Data interaction between the doctor program controller and the stimulator is not limited in the embodiments of the present application. When the doctor performs remote program control, the doctor program controller may perform the data interaction with the stimulator via a server and the patient program controller. When the doctor performs offline program control with the patient face to face, the doctor program controller may perform data interaction with the stimulator via the patient program controller and may also directly perform data interaction with the stimulator.

The patient program controller may include a host (in communication with the server) and a slave (in communication with the stimulator), and the host can be communicatively connected to the slave. The doctor program controller may perform data interaction with the server via a 3rd-generation (3G)/4th-generation (4G)/5th-generation (5G) network. The server may perform data interaction with the host via the 3G/4G/5G network. The host may perform data interaction with the slave via a Bluetooth protocol/Wi-Fi protocol/Universal Serial Bus (USB) protocol. The slave may perform data interaction with the stimulator via an operation frequency band of 401 MHz to 406 MHz/operation frequency band of 2.4 GHz to 2.48 GHz. The doctor program controller may directly perform data interaction with the stimulator via the operation frequency band of 401 MHz to 406 MHz/operation frequency band of 2.4 GHz to 2.48 GHz.

In addition to the application field of the preceding implantable devices, the embodiments of the present application may also be applied to the technical fields of non-implantable medical devices and even non-medical devices, which is not limited in the embodiments of the present application as long as real-time state diagnoses to update the stimulation parameters are involved.

### [System embodiment]

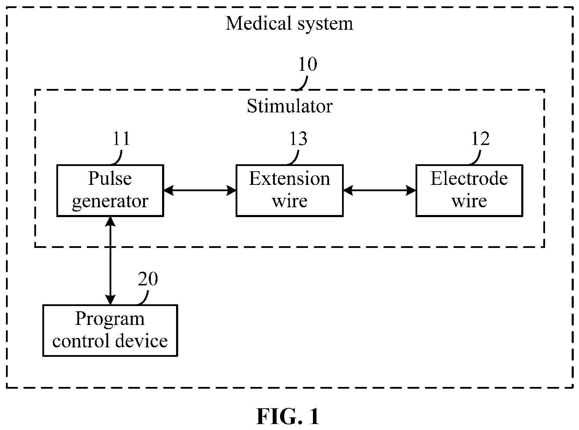

Referring to FIG. 1, FIG. 1 is a block diagram of a medical system according to an embodiment of the present application.

An embodiment of the present application provides a medical system. The medical system includes a pulse generator 11, an electrode wire 12, and a program control device 20. The electrode wire 12 is configured to sense the electrophysiological activity of a patient and deliver electrical stimulation. The program control device 20 is configured to establish a program control connection with the pulse generator 11, receive a range configuration operation, and determine an adjustable numerical range corresponding to each stimulation parameter of the pulse generator 11 in response to the range configuration operation. The pulse generator 11 is configured to perform adaptive adjustment within the adjustable numerical range corresponding to each stimulation parameter.

Thus, a doctor may configure the numerical range that allows the pulse generator 11 to adaptively adjust each stimulation parameter to ensure the safety of an electrical stimulation therapy. The doctor may configure different numerical ranges for different patients according to the severity of their illnesses, taking into account both safety and treatment effect.

In the embodiment of the present application, the medical system may further include an extension wire 13, and the pulse generator 11, the electrode wire 12, and the extension wire 13 form a stimulator 10. When the pulse generator 11 and the electrode wire 12 are both implanted into the skull of the patient, the stimulator 10 may also exclude the extension wire 13 but only include the pulse generator 11 and the electrode wire 12.

In the embodiment of the present application, the pulse generator 11 and the electrode wire 12 may be communicatively connected. The pulse generator 11 and the electrode wire 12 may directly communicate with each other or may also exchange data through the extension wire 13.

In the embodiment of the present application, the electrode wire 12 may be implanted into the skull of the patient or other locations of the patient in vivo. The number of electrode wires 12 may be, for example, 1, 2, 3, 4, 5, or 6. The number of electrode contacts of each electrode wire 12 may be, for example, 4, 6, 8, 9, 10, 12, 15, or 18. When implanted into the skull of the patient, multiple electrode wires 12 may be implanted into the same hemisphere of the brain or may also be implanted into the two hemispheres of the brain respectively.

The program control device 20 is not limited in the embodiment of the present application. The program control device 20 may include, for example, one or more of a tablet computer, a laptop computer, a desktop computer, a mobile phone, or a smart wearable device, and may also be a console or a workstation.

A manner of using the program control device 20 to receive the range configuration operation is not limited in the present application. Operations are divided according to input manners and may include, for example, a text input operation, an audio input operation, a video input operation, a key operation, a mouse operation, a keyboard operation, and a smart stylus operation.

In some embodiments, the disease of the patient includes at least one of seizure disorder, depressive disorder, bipolar disorder, anxiety disorder, post-traumatic stress disorder, obsessive-compulsive disorder, behavioral disorder, mood disorder, memory disorder, mental state disorder, tremor, Parkinson's disease, Huntington's disease, Alzheimer's disease, addictive disorder, or autism.

In the embodiment of the present application, the pulse generator 10 may be configured to execute steps of an adaptive stimulation method. The adaptive stimulation method is described first hereinafter, and then the pulse generator 10 is described.

### [Method embodiment]

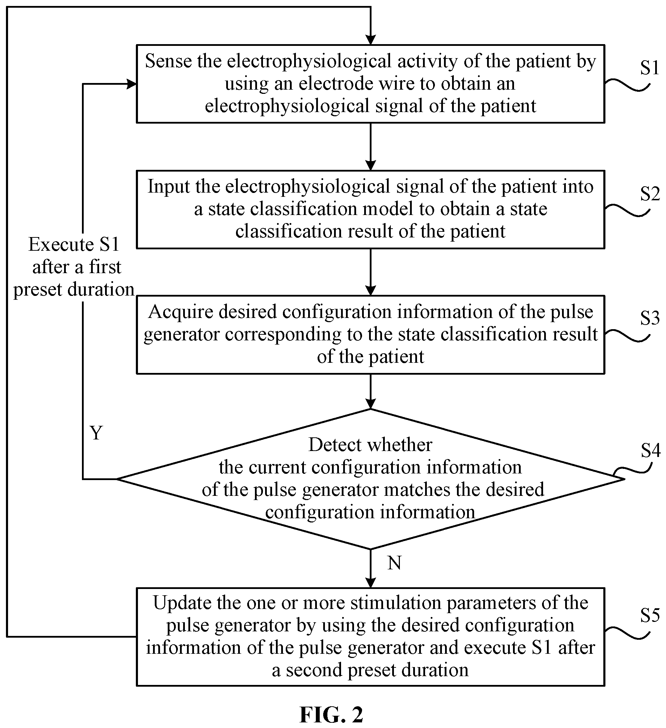

Referring to FIG. 2, FIG. 2 is a flowchart of an adaptive stimulation method according to an embodiment of the present application.

The present application further provides an adaptive stimulation method for adaptively adjusting one or more stimulation parameters of a pulse generator, where the pulse generator is implanted into a patient. The method includes the steps below.

In S1, the electrophysiological activity of the patient is sensed by using an electrode wire to obtain an electrophysiological signal of the patient.

In S2, the electrophysiological signal of the patient is input into a state classification model to obtain a state classification result of the patient.

In S3, desired configuration information of the pulse generator corresponding to the state classification result of the patient is acquired.

In S4, whether the current configuration information of the pulse generator matches the desired configuration information is detected; if the current configuration information of the pulse generator does not match the desired configuration information, S5 is executed; and if the current configuration information of the pulse generator matches the desired configuration information, the method returns to execute S1 after a first preset duration.

In S5, the one or more stimulation parameters of the pulse generator are updated by using the desired configuration information of the pulse generator, and the method returns to execute S1 after a second preset duration.

The second preset duration is less than the first preset duration.

Thus, the electrophysiological activity of the patient is sensed in real time by using the electrode wire to obtain the real-time electrophysiological signal of the patient; the states of the sensed electrophysiological signal classified by using the state classification model to obtain the real-time state classification result of the patient intelligently and automatically; the desired configuration information of the corresponding pulse generator is acquired intelligently and automatically based on the real-time state classification result of the patient; the current configuration information of the pulse generator is compared with the desired configuration information to determine whether the current configuration information matches the desired configuration information, if the current configuration information matches the desired configuration information, there is no need to adjust the one or more stimulation parameters of the pulse generator, and if the current configuration information does not match the desired configuration information, it is necessary to adjust the one or more stimulation parameters of the pulse generator so that the pulse generator can deliver electrical stimulation with a stimulation parameter corresponding to the desired configuration information to the patient via electrode contacts of the electrode wire; regardless of whether parameter adjustment is performed, the step of sensing the electrophysiological activity of the patient (that is, S1) is executed again after a certain duration, thereby forming a cyclical process of [sensing, state classification, acquiring the desired configuration information, configuration information comparison, adjustment/non-adjustment, sensing, state classification, acquiring the desired configuration information, configuration information comparison, adjustment/non-adjustment, ...] and fulfilling the adaptive stimulation function based on state diagnoses.

In the two cases of adjusting/not adjusting the one or more stimulation parameters, the interval durations to the next sensing step are different. If the one or more stimulation parameters are adjusted, the interval duration (the second preset duration) is short, and if the one or more stimulation parameters do not need to be adjusted, the interval duration is long (the first preset duration).

The advantage of this is that when the real-time state of the patient changes, there may be frequent changes in the state. In the case where the state frequent changes, if a long interval is maintained for real-time sensing, the configuration of the one or more stimulation parameters cannot quickly keep up with the change in the state of the patient. Therefore, the interval duration needs to be shortened.

Once the one or more stimulation parameters of the patient are adjusted, the next sensing step is executed at a short interval duration to solve the problem that one adjustment may not be sufficient to meet actual requirements.

In addition, in the case where the real-time state of the patient remains unchanged, and there is no need to adjust the one or more stimulation parameters, a long interval duration is maintained before executing the next sensing step so that the amount of data calculation of the pulse generator can be reduced, thereby reducing the power consumption of the pulse generator, (when the pulse generator is rechargeable) prolonging the time interval between two charges of the pulse generator and prolonging the service life of components of the pulse generator.

In the embodiment of the present application, the electrode wire may be configured to sense the electrophysiological activities of a single cell and/or multiple cells to obtain an electrophysiological signal of the single cell and/or a local field potential of the single cell. The local field potential (LFP) is a special electrophysiological signal. In an organism in vivo, the synaptic activity of dendrites in biological tissue with a certain volume induces a current. When this current flows through the extracellular space with a certain impedance, a certain voltage distribution is formed. A local voltage value recorded at a point is referred to as the local field potential.

The state classification result of the patient is not limited in the embodiment of the present application and may be classified as, for example, a controlled condition or an uncontrolled condition; onset, imminent onset, or not onset; no symptom, a mild symptom, a moderate symptom, or a severe symptom; sleep or exercise; or happiness, sadness, or anxiety. For different status classification results, corresponding desired configuration information is often different.

The one or more stimulation parameters of the pulse generator may include at least one of a frequency (for example, the number of electrical stimulation pulse signals within a unit time of 1 s, in Hz), a pulse width (the duration of each pulse, in µs), an amplitude (generally represented by voltage, that is, the strength of each pulse, in V), a timing (for example, may be continuous or triggered), a stimulation mode (including one or more of a current mode, a voltage mode, a timing stimulation mode, or a cyclical stimulation mode), doctor-controlled upper and lower limits (a range that can be adjusted by a doctor), or patient-controlled upper and lower limits (a range that can be autonomously adjusted by a patient).

In an application scenario, the stimulation parameters of the stimulator may be adjusted in the current mode or the voltage mode.

In the embodiment of the present application, the current configuration information of the pulse generator matching the desired configuration information means that the current configuration information is completely consistent with the desired configuration information; or the difference between the current configuration information and each stimulation parameter in the desired configuration information is not greater than a preset threshold value corresponding to each stimulation parameter; or the similarity between the current configuration information and the desired configuration information is not less than a preset similarity threshold. The preset similarity threshold may be, for example, 85%, 89%, 96%, or 99%.

In an application scenario, the current configuration information of the pulse generator is [for a stimulation pulse signal, the voltage amplitude is 4.9 V, the pulse width is 60 µs, and the frequency is 130 Hz], and the desired configuration information is [for a stimulation pulse signal, the voltage amplitude is 4.9 V, the pulse width is 60 µs, and the frequency is 130 Hz], so the current configuration information matches the desired configuration information.

In another application scenario, the current configuration information of the pulse generator is [for a stimulation pulse signal, the voltage amplitude is 3 V, the pulse width is 30 µs, and the frequency is 100 Hz], and the desired configuration information is [for a stimulation pulse signal, the voltage amplitude is 4.9 V, the pulse width is 60 µs, and the frequency is 130 Hz], so the current configuration information does not match the desired configuration information.

In still another application scenario, the current configuration information of the pulse generator is [for a stimulation pulse signal, the voltage amplitude is 4.8 V, the pulse width is 58 µs, and the frequency is 127 Hz], and the desired configuration information is [for a stimulation pulse signal, the voltage amplitude is 4.9 V, the pulse width is 60 µs, and the frequency is 130 Hz], so the current configuration information matches the desired configuration information.

The first preset duration and the second preset duration are not limited in the embodiment of the present application, as long as the first preset duration is greater than the second preset duration.

In some embodiments, the first preset duration may be, for example, 5 minutes, 10 minutes, 15 minutes, 30 minutes, 1 hour, or 2 hours, and the second preset duration may be, for example, 5 seconds, 10 seconds, 15 seconds, 30 seconds, 1 minute, 2 minutes, or 3 minutes.

In some embodiments, the training process of the state classification model may include: acquiring a first training set, where the first training set includes multiple first training data, and each piece of first training data includes an electrophysiological signal of a first sample object and annotated data of a state classification result of the first sample object; and performing the following processing for each piece of first training data in the first training set: inputting the electrophysiological signal of the first sample object in each piece of first training data into a preset first deep learning model to obtain predicted data of the state classification result of the first sample object; updating a model parameter of the first deep learning model according to the predicted data of the state classification result of the first sample object and the annotated data of the state classification result of the first sample object; detecting whether a preset first training end condition is satisfied; if the preset first training end condition is satisfied, using the trained first deep learning model as the state classification model; and if the preset first training end condition is not satisfied, continuing training the first deep learning model by using the next piece of first training data.

Thus, the appropriate number of neuron computing nodes and a multi-layer computational hierarchy are established through a design, and an appropriate input layer and output layer are selected to obtain the preset first deep learning model. A functional relationship between input and output is established through the learning and optimization of the first deep learning model. Although the functional relationship between input and output cannot be 100% found, a realistic correlation can be approached as closely as possible. The state classification model trained in this manner may acquire the (real-time) state classification result of the patient according to the electrophysiological signal of the patient (sensed in real time), and the accuracy and reliability of the calculation result are high.

In some embodiments, the present application may train the state classification model, and a parameter configuration model and a duration calculation model described later. In some other embodiments, the present application may use a pre-trained state classification model, a pre-trained parameter configuration model, and a pre-trained duration calculation model.

In some embodiments, for example, data mining may be performed on historical data to acquire relevant data (electrophysiological signals and state classification results) of sample objects (including the first sample object, a second sample object described later, and a third sample object described later) in training sets (including the first training set, a second training set described later, and a third training set described later) and relevant data (power) of sample pulse generators. In other words, these sample objects may be real patients, and the sample pulse generators may be real pulse generators. The relevant data of the sample objects and the relevant data of the sample pulse generators may also be automatically generated by a generation network of a generative adversarial network (GAN) model.

The GAN model consists of one generation network and one discrimination network. The generation network randomly samples from the latent space as the input, and the output of the generation network needs to imitate a real sample in the training sets as much as possible. The input of the discrimination network is a real sample or the output of the generation network so as to distinguish the output of the generation network from the real sample as much as possible. The generation network should deceive the discrimination network as much as possible. The two networks compete with each other and constantly adjust parameters so as to finally make the discrimination network unable to determine whether the output of the generation network is real. The GAN model may generate the relevant data of the multiple sample objects and the relevant data of the multiple sample pulse generators for the training processes of models, which can effectively reduce the amount of original data collection and greatly reduce the cost of data collection and annotation.

A manner of acquiring the annotated data is not limited in the present application and may be, for example, a manual annotation manner, an automatic annotation manner, or a semi-automatic annotation manner. When the sample objects are the real patients, and the sample pulse generators are the real pulse generators, real data may be acquired from the historical data as the annotated data in a manner of keyword extraction.

The training processes of the state classification model, the parameter configuration model, and the duration calculation model are not limited in the present application and may be, for example, performed in a supervised learning training manner, a semi-supervised learning training manner, or an unsupervised learning training manner.

Preset training end conditions (including the first training end condition, a second training end condition described later, and a third training end condition described later) are not limited in the present application and may be, for example, that the number of times of training reaches a preset number (the preset number is, for example, 1, 3, 10, 100, 1000, or 10000), all training data in the training sets are trained one or more times, or a total loss value obtained after the training is not greater than a preset loss value.

In some embodiments, S3 may further include:
inputting the state classification result of the patient into the parameter configuration model to obtain the desired configuration information of the pulse generator, where the training process of the parameter configuration model includes: acquiring a second training set, where the second training set includes multiple second training data, and each piece of second training data includes a state classification result of a second sample object and annotated data of the desired configuration information of the pulse generator; and performing the following processing for each piece of second training data in the second training set: inputting the state classification result of the second sample object in each piece of second training data into a preset second deep learning model to obtain predicted data of the desired configuration information of the pulse generator; updating a model parameter of the second deep learning model according to the predicted data of the desired configuration information of the pulse generator and the annotated data of the desired configuration information of the pulse generator; detecting whether a preset second training end condition is satisfied; if the preset second training end condition is satisfied, using the trained second deep learning model as the parameter configuration model; and if the preset second training end condition is not satisfied, continuing training the second deep learning model by using the next piece of second training data.

Thus, the appropriate number of neuron computing nodes and a multi-layer computational hierarchy are established through a design, and an appropriate input layer and output layer are selected to obtain the preset second deep learning model. A functional relationship between input and output is established through the learning and optimization of the second deep learning model. Although the functional relationship between input and output cannot be 100% found, a realistic correlation can be approached as closely as possible. The parameter configuration model trained in this manner may acquire the desired configuration information of the pulse generator according to the state classification result of the patient, and the accuracy and reliability of the calculation result are high.

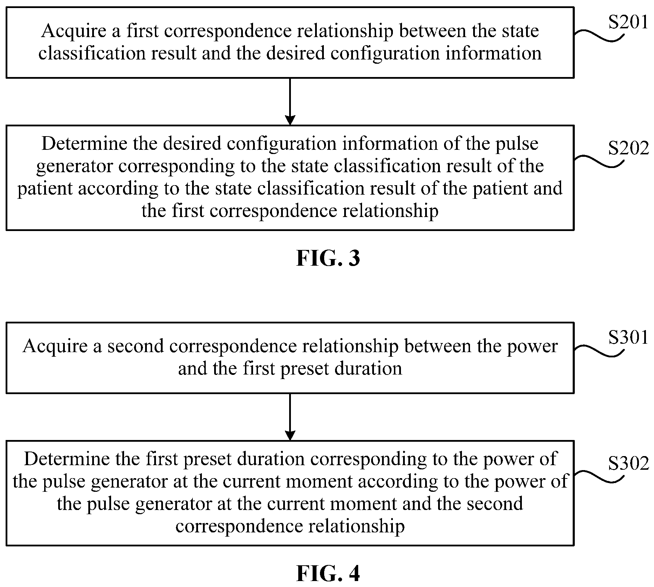

Referring to FIG. 3, FIG. 3 is a flowchart of acquiring desired configuration information according to an embodiment of the present application.In some embodiments, S3 may further include the steps below.

In S201, a first correspondence relationship between the state classification result and the desired configuration information is acquired.

In S202, the desired configuration information of the pulse generator corresponding to the state classification result of the patient is determined according to the state classification result of the patient and the first correspondence relationship.

Thus, the first correspondence relationship between the state classification result and the desired configuration information is pre-established, and the first correspondence relationship may be used for acquiring the desired configuration information corresponding to the state classification result of the patient. The desired configuration information of the pulse generator is acquired by using the preceding manner. Consequently, the calculation process is simple, the amount of calculation is small, the calculation speed is fast, and the consumption of calculation resources is small. When there are only a few state classification results for the patient, the preceding manner has a more prominent advantage, and desired configuration information corresponding to each state classification result can be quickly obtained.

In some embodiments, the desired configuration information of the pulse generator may be configured to indicate a parameter value of each of one or more of the following stimulation parameters: the voltage amplitude of a stimulation pulse signal, the pulse width of the stimulation pulse signal, and the frequency of the stimulation pulse signal; or identification information of an electrode contact for delivering electrical stimulation.

Thus, the electrode wire is provided with the multiple electrode contacts, which can not only adjust the electrical stimulation energy released by an electrode contact that is performing a stimulation task (by adjusting the voltage amplitude of the stimulation pulse signal, the pulse width of the stimulation pulse signal, and the frequency of the stimulation pulse signal), but also select an appropriate electrode contact to perform the stimulation task.

The advantage of this is that, for example, according to the strength of the sensed electrophysiological signal, the one or more stimulation parameters can be automatically adjusted, or the electrode contacts for delivering the electrical stimulation can be automatically replaced. On the basis of ensuring therapeutic efficacy, the energy (power consumption) can be saved, the service life of the pulse generator can be prolonged, and the financial burden on the patient can be alleviated, thereby helping improve the acceptance of the implantable medical apparatus of the patient and the enthusiasm of the patient for using the implantable medical apparatus and helping widen the market prospect of the implantable medical apparatus.

The identification information is not limited in the embodiment of the present application and may be, for example, represented by one or more of Chinese characters, letters, numbers, or symbols.

In an application scenario, the desired configuration information of the pulse generator is [for a stimulation pulse signal, the voltage amplitude is 4.9 V, the pulse width is 60 µs, the frequency is 130 Hz, and the identification information of the electrode contact for delivering the electrical stimulation is 3# and 6#].

In some embodiments, the acquiring process of the first preset duration may include: inputting the state classification result of the patient and the power of the pulse generator into the duration calculation model to obtain the first preset duration, where the training process of the duration calculation model includes: acquiring a third training set, where the third training set includes multiple third training data, and each piece of third training data includes a state classification result of a third sample object, the power of a sample pulse generator, and annotated data of the first preset duration; and performing the following processing for each piece of third training data in the third training set: inputting the state classification result of the third sample object in each piece of third training data and the power of the sample pulse generator into a preset third deep learning model to obtain predicted data of the first preset duration; updating a model parameter of the third deep learning model according to the predicted data of the first preset duration and the annotated data of the first preset duration; detecting whether a preset third training end condition is satisfied; if yes, using the trained third deep learning model as the duration calculation model; and if not, continuing training the third deep learning model by using the next piece of third training data.

Thus, the appropriate number of neuron computing nodes and a multi-layer computational hierarchy are established through a design, and an appropriate input layer and output layer are selected to obtain the preset third deep learning model. A functional relationship between input and output is established through the learning and optimization of the third deep learning model. Although the functional relationship between input and output cannot be 100% found, a realistic correlation can be approached as closely as possible. The duration calculation model trained in this manner may acquire the first preset duration to the next sensing step according to the state classification result of the patient (acquired in real time) and the power of the pulse generator at the current moment, and the accuracy and reliability of the calculation result are high.

In addition, dynamically adjusting the first preset duration according to the condition of the patient and the power of the pulse generator is more in line with the requirements of actual applications than configuring the first preset duration to a fixed value.

For example, when the patient is in an onset state, and the pulse generator has sufficient power, quickly controlling the condition of the patient is the top priority, and the speed of power consumption is a secondary concern, so the corresponding first preset duration may be short (for example, 10 minutes), and shortening the interval between two state diagnoses helps promptly discover a situation where a parameter configuration needs to be adjusted. Moreover, when the patient is not in an onset state, and the pulse generator has very low power, to avoid interrupting an electrical stimulation therapy and affecting the condition control of the patient due to the shutting down of the pulse generator caused by the depletion of power, a long first preset duration (for example, 30 minutes) may be configured to prolong the battery life of the pulse generator.

The pulse generator in the embodiment of the present application may be, for example, a rechargeable pulse generator or a non-rechargeable pulse generator. When the rechargeable pulse generator is used, the use of the preceding manner of dynamically adjusting the first preset duration can reduce the number of times of charging the pulse generator.

Referring to FIG. 4, FIG. 4 is a flowchart of acquiring a first preset duration according to an embodiment of the present application. In some embodiments, the acquiring process of the first preset duration may include the steps below.

In S301, a second correspondence relationship between the power and the first preset duration is acquired.

In S302, the first preset duration corresponding to the power of the pulse generator at the current moment is determined according to the power of the pulse generator at the current moment and the second correspondence relationship.

Thus, the second correspondence relationship between the power and the first preset duration is pre-established, and the second correspondence relationship may be used for acquiring the first preset duration corresponding to the power of the pulse generator at the current moment. For example, when the power is high, a short first preset duration (for example, 10 minutes) may be used, and when the power is low, a long first preset duration (for example, 30 minutes) may be used. The preceding manner is used for acquiring the first preset duration. Consequently, the calculation process is simple, the amount of calculation is small, the calculation speed is fast, and the consumption of calculation resources is small.

In the embodiment of the present application, both the first correspondence relationship and the second correspondence relationship may be in the form of a comparison table, and the comparison table is pre-stored locally in the pulse generator. In some embodiments, the disease of the patient may include at least one of: seizure disorder, depressive disorder, bipolar disorder, anxiety disorder, post-traumatic stress disorder, obsessive-compulsive disorder, behavioral disorder, mood disorder, memory disorder, mental state disorder, tremor, Parkinson's disease, Huntington's disease, Alzheimer's disease, addictive disorder, or autism.

Thus, in the electrical stimulation therapy, electrical stimulation is directly performed on neural targets (for example, tissue, nuclei, and fiber bundles, such as nucleus accumbens, anterior limbs of the internal capsule, caudate nuclei, lenticular nuclei, and putamen), which can effectively control the conditions of the preceding diseases, alleviate the symptoms of patients, and relieve the pain of the patients.

In an application scenario, the electrode wire is implanted into the skull of the patient, and all the electrode contacts of the electrode wire have the functions of sensing an electrophysiological activity and delivering electrical stimulation; the pulse generator can use the electrode wire to sense the local field potential of the brain of the patient, and when the patient is an imminent onset state or an onset state, the pulse generator can send precise and intermittent stimulation signals to the brain of the patient in an intelligent and automatic manner.

When the patient is postoperatively monitored to be in a state of poorly controlled symptoms (the state classification result is that the condition is not controlled), the pulse generator automatically starts the stimulation mode.

When the patient remains in a state of controlled symptoms for a long time (the state classification result is that the condition is controlled), the pulse generator automatically reduces the one or more stimulation parameters or even stops the electrical stimulation therapy.

The pulse generator may also automatically adjust the one or more stimulation parameters or replace the electrode contacts according to the strength of the sensed electrophysiological signal so that on the basis of ensuring the therapeutic efficacy, the power can be saved, the service life of the pulse generator can be prolonged, and the financial burden on the patient can be alleviated.

The preceding state classification model, parameter configuration model, and duration calculation model can be established by accumulating a certain amount of the relevant data and analyzing the specific electrophysiological signal that reflects the state of the patient, and brain nerves of the patient are precisely stimulated by using artificial intelligence technologies such as deep learning and adaptive learning, which reduces doctor manual intervention and adjustment and accurately controls the symptoms of the patient.

In some other embodiments, the desired configuration information of the pulse generator may be acquired based on the electrophysiological signal of the patient and disease information of the patient. The desired configuration information obtained based on the patient's age, gender, disease type, medical history, treatment records, and program control records can comprehensively consider various factors affecting the treatment effect so that the accuracy can be higher, and the treatment effect can be improved.

The disease information is not limited in the embodiment of the present application and may include, for example, one or more of the patient's basic information, medical history information, medical imaging information, pre-recorded video information, real-time video information, the program control records, or audio and video records. The patient's basic information may include the patient's age, gender, and disease type.

The medical imaging information in the embodiment of the present application may include, for example, computed tomography (CT) data, magnetic resonance (MR) data, positron emission tomography (PET) data, X-ray data, PET-CT data, and PET-MR data. Correspondingly, a medical scanning device used may be, for example, a CT device, an MR device, a PET device, an X-ray device, a PET-CT device, or a PET-MR device.

### [Apparatus embodiment]

An embodiment of the present application further provides a pulse generator. Specific implementations of the pulse generator are the same as the implementations that are recorded in the preceding method embodiment and the technical effects that are achieved in the preceding method embodiment, and some contents are not repeated.

The present application further provides the pulse generator. The pulse generator is implanted into a patient and includes a processor. The processor is configured to: (S1) sense the electrophysiological activity of the patient by using an electrode wire to obtain an electrophysiological signal of the patient; (S2) input the electrophysiological signal of the patient into a state classification model to obtain a state classification result of the patient; (S3) acquire desired configuration information of the pulse generator corresponding to the state classification result of the patient; (S4) detect whether the current configuration information of the pulse generator matches the desired configuration information, if not, execute S5, and if yes, execute S1 after a first preset duration; and (S5) update one or more stimulation parameters of the pulse generator by using the desired configuration information of the pulse generator and execute S1 after a second preset duration, where the second preset duration is less than the first preset duration.

In some embodiments, the training process of the state classification model may include: acquiring a first training set, where the first training set includes multiple first training data, and each piece of first training data includes an electrophysiological signal of a first sample object and annotated data of a state classification result of the first sample object; and performing the following processing for each piece of first training data in the first training set: inputting the electrophysiological signal of the first sample object in each piece of first training data into a preset first deep learning model to obtain predicted data of the state classification result of the first sample object; updating a model parameter of the first deep learning model according to the predicted data of the state classification result of the first sample object and the annotated data of the state classification result of the first sample object; detecting whether a preset first training end condition is satisfied; if the preset first training end condition is satisfied, using the trained first deep learning model as the state classification model; and if the preset first training end condition is not satisfied, continuing training the first deep learning model by using the next piece of first training data.

In some embodiments, the processor may be configured to acquire the desired configuration information of the pulse generator corresponding to the state classification result of the patient in the following manner: inputting the state classification result of the patient into a parameter configuration model to obtain the desired configuration information of the pulse generator, where the training process of the parameter configuration model includes: acquiring a second training set, where the second training set includes multiple second training data, and each piece of second training data includes a state classification result of a second sample object and annotated data of the desired configuration information of the pulse generator; and performing the following processing for each piece of second training data in the second training set: inputting the state classification result of the second sample object in each piece of second training data into a preset second deep learning model to obtain predicted data of the desired configuration information of the pulse generator; updating a model parameter of the second deep learning model according to the predicted data of the desired configuration information of the pulse generator and the annotated data of the desired configuration information of the pulse generator; detecting whether a preset second training end condition is satisfied; if the preset second training end condition is satisfied, using the trained second deep learning model as the parameter configuration model; and if the preset second training end condition is not satisfied, continuing training the second deep learning model by using the next piece of second training data.

In some embodiments, the processor may be configured to acquire the desired configuration information of the pulse generator corresponding to the state classification result of the patient in the following manner: acquiring a first correspondence relationship between the state classification result and the desired configuration information; and determining the desired configuration information of the pulse generator corresponding to the state classification result of the patient according to the state classification result of the patient and the first correspondence relationship.

In some embodiments, the desired configuration information of the pulse generator may be configured to indicate a parameter value of each of one or more of the following stimulation parameters: the voltage amplitude of a stimulation pulse signal, the pulse width of the stimulation pulse signal, and the frequency of the stimulation pulse signal; or identification information of an electrode contact for delivering electrical stimulation.

In some embodiments, the acquiring process of the first preset duration may include: inputting the state classification result of the patient and the power of the pulse generator into a duration calculation model to obtain the first preset duration, where the training process of the duration calculation model includes: acquiring a third training set, where the third training set includes multiple third training data, and each piece of third training data includes a state classification result of a third sample object, the power of a sample pulse generator, and annotated data of the first preset duration; and performing the following processing for each piece of third training data in the third training set: inputting the state classification result of the third sample object in each piece of third training data and the power of the sample pulse generator into a preset third deep learning model to obtain predicted data of the first preset duration; updating a model parameter of the third deep learning model according to the predicted data of the first preset duration and the annotated data of the first preset duration; detecting whether a preset third training end condition is satisfied; if the preset third training end condition is satisfied, using the trained third deep learning model as the duration calculation model; and if the preset third training end condition is not satisfied, continuing training the third deep learning model by using the next piece of third training data.

In some embodiments, the acquiring process of the first preset duration may include: acquiring a second correspondence relationship between the power and the first preset duration; and determining the first preset duration corresponding to the power of the pulse generator at the current moment according to the power of the pulse generator at the current moment and the second correspondence relationship.

In some embodiments, the disease of the patient may include at least one of seizure disorder, depressive disorder, bipolar disorder, anxiety disorder, post-traumatic stress disorder, obsessive-compulsive disorder, behavioral disorder, mood disorder, memory disorder, mental state disorder, tremor, Parkinson's disease, Huntington's disease, Alzheimer's disease, addictive disorder, or autism.

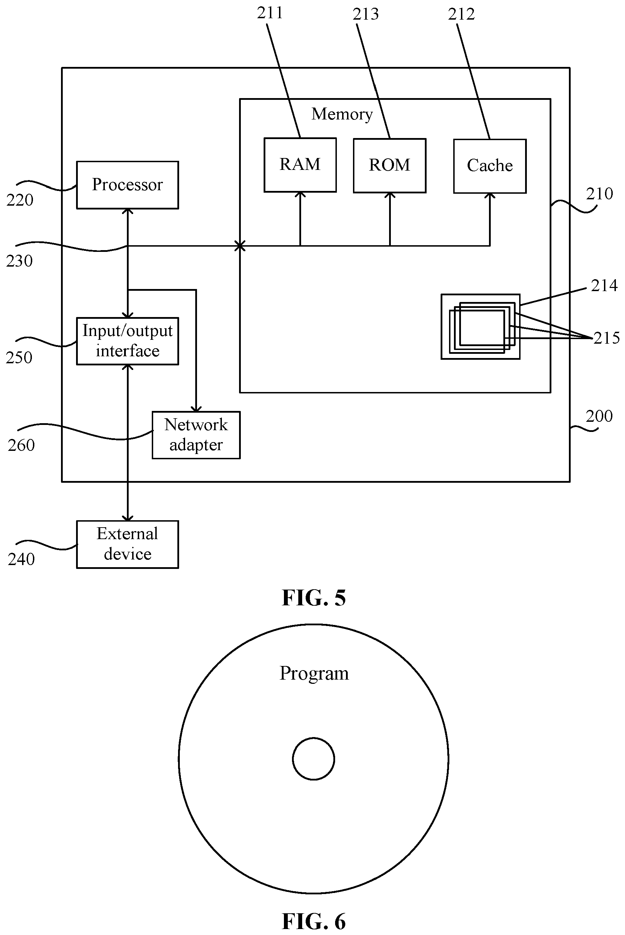

Referring to FIG. 5, FIG. 5 is a block diagram of a pulse generator according to an embodiment of the present application.

The pulse generator 200 may include, for example, at least one memory 210, at least one processor 220, and a bus 230 connected to different platform systems.

The at least one memory 210 may include a readable medium in the form of a volatile memory, such as a random-access memory (RAM) 211 and/or a cache 212, and may further include a read-only memory (ROM) 213.

The at least one memory 210 also stores a computer program which may be executed by the at least one processor 220 so that the at least one processor 220 can fulfill the functions of the preceding pulse generator. Specific implementations of the functions are the same as the implementations that are recorded in the preceding method embodiment and the technical effects that are achieved in the preceding method embodiment, and some contents are not repeated.

The at least one memory 210 may further include a utility 214 having at least one program module 215. Such program modules 215 include an operating system, one or more application programs, and other program modules and program data. Each or a combination of these examples may include the implementation of a network environment.

Correspondingly, the at least one processor 220 may execute the preceding computer program and may execute the utility 214. The at least one processor 220 may use one or more application-specific integrated circuits (ASICs), digital signal processors (DSPs), programmable logic devices (PLDs), complex programmable logic devices (CPLDs), field-programmable gate arrays (FPGAs), or other electronic elements.

The bus 230 may be a local bus representing one or more of several types of bus structures, including a memory bus or memory controller, a peripheral bus, an accelerated graphics port, a processor, or using any one of a variety of bus structures.

The pulse generator 200 may also communicate with one or more external devices 240 such as a keyboard, a pointing device, or a Bluetooth device. The pulse generator 200 may further communicate with one or more devices that can interact with the pulse generator 200, and/or with any device (such as a router or a modem) that enables the pulse generator 200 to communicate with one or more other computing devices. These communications may be performed through an input/output interface 250. Moreover, the pulse generator 200 may further communicate with one or more networks (such as a local area network (LAN), a wide area network (WAN) and/or a public network such as the Internet) through a network adapter 260. The network adapter 260 may communicate with other modules of the pulse generator 200 via the bus 230. Although not shown in the figures, other hardware and/or software modules may be used in conjunction with the pulse generator 200. The other hardware and/or software modules include a microcode, a device driver, a redundant processor, an external disk drive array, a redundant array of independent disks (RAID) system, a tape driver, and a data backup storage system.

### [Medium embodiment]

An embodiment of the present application further provides a computer-readable storage medium storing a computer program. The computer program, when executed by a processor, causes the processor to fulfill the functions of the preceding pulse generator or execute the steps of any one of the preceding methods. Specific implementations of the functions and the steps are the same as the implementations that are recorded in the preceding method and the technical effects that are achieved in the preceding method, and some contents are not repeated.

Referring to FIG. 6, FIG. 6 is a diagram illustrating the structure of a program product according to an embodiment of the present application.

The program product may use a portable compact disk read-only memory (CD-ROM), may include a program code, and may run on terminal devices such as a personal computer. However, the program product of the present application is not limited thereto. In the embodiment of the present application, the readable storage medium may be any tangible medium including or storing a program. The program may be used by or used in conjunction with an instruction execution system, apparatus, or element. The program product may use one readable medium or any combination of multiple readable media. The readable medium may be a readable signal medium or a readable storage medium. The readable storage medium may be, for example, an electronic, magnetic, optical, electromagnetic, infrared, or semiconductor system, apparatus, or device, or any combination thereof. Examples (a non-exhaustive list) of the readable storage medium include an electrical connection having one or more wires, a portable disk, a hard disk, a RAM, a ROM, an erasable programmable read-only memory (EPROM), a flash memory, an optical fiber, a CD-ROM, an optical storage device, a magnetic storage device, or any suitable combination thereof.

The computer-readable storage medium may include a data signal propagated in a baseband or as part of a carrier. Readable program codes are carried in the data signal. This propagated data signal may take multiple forms including an electromagnetic signal, an optical signal, or any suitable combination thereof. The readable storage medium may also be any readable medium, and the readable medium may send, propagate or transmit a program used by or in combination with an instruction execution system, apparatus or device. The program codes included in the readable storage medium may be transmitted using any appropriate medium, including wireless, wireline, fiber-optic cable, Radio Frequency (RF), or any appropriate combination thereof. The program codes for performing the operations of the present invention may be written in one programming language or any combination of multiple programming languages. The programming languages include object-oriented programming languages such as Java and C++ and further include conventional procedural programming languages such as C programming language or similar programming languages. The program codes may be executed entirely on a user computing device, executed partly on a user computing device, executed as a stand-alone software package, executed partly on a user computing device and partly on a remote computing device, or executed entirely on a remote computing device or a server. In the scenario involving the remote computing device, the remote computing device may be connected to the user computing device through any type of network, including a LAN or a WAN, or may be connected to an external computing device (for example, through the Internet using an Internet service provider).

## Claims

1. A pulse generator, the pulse generator (11) being implanted into a patient and comprising a processor, wherein the processor is configured to perform the following operations:
sensing an electrophysiological activity of the patient by using an electrode wire (12) to obtain an electrophysiological signal of the patient;
inputting the electrophysiological signal of the patient into a state classification model to obtain a state classification result of the patient;
acquiring desired configuration information of the pulse generator (11) corresponding to the state classification result of the patient;
detecting whether current configuration information of the pulse generator (11) matches the desired configuration information;
in response to the current configuration information of the pulse generator (11) not matching the desired configuration information, updating at least one stimulation parameter of the pulse generator (11) by using the desired configuration information of the pulse generator (11), and after a second preset duration, returning to perform the operation of sensing the electrophysiological activity of the patient by using the electrode wire (12) to obtain the electrophysiological signal of the patient; and
in response to the current configuration information of the pulse generator (11) matching the desired configuration information, after a first preset duration, returning to perform the operation of sensing the electrophysiological activity of the patient by using the electrode wire (12) to obtain the electrophysiological signal of the patient,
wherein the second preset duration is less than the first preset duration.

2. The pulse generator of claim 1, wherein a training process of the state classification model comprises:
acquiring a first training set, wherein the first training set comprises a plurality of pieces of first training data, and each piece of first training data among the plurality of pieces of first training data comprises an electrophysiological signal of a first sample object and annotated data of a state classification result of the first sample object; and
for the each piece of first training data in the first training set, performing the following processing:
inputting the electrophysiological signal of the first sample object in the each piece of first training data into a preset first deep learning model to obtain predicted data of the state classification result of the first sample object;
updating a model parameter of the first deep learning model according to the predicted data of the state classification result of the first sample object and the annotated data of the state classification result of the first sample object; and
detecting whether a preset first training end condition is satisfied; in response to satisfying the first training end condition, using the trained first deep learning model as the state classification model; and in response to not satisfying the first training end condition, continuing training the first deep learning model by using next piece of first training data.

3. The pulse generator of claim 1, wherein the processor is configured to acquire the desired configuration information of the pulse generator (11) corresponding to the state classification result of the patient in the following manner:
inputting the state classification result of the patient into a parameter configuration model to obtain the desired configuration information of the pulse generator (11),
wherein a training process of the parameter configuration model comprises:
acquiring a second training set, wherein the second training set comprises a plurality of pieces of second training data, and each piece of second training data among the plurality of pieces of second training data comprises a state classification result of a second sample object and annotated data of the desired configuration information of the pulse generator (11); and
for the each piece of second training data in the second training set, performing the following processing:
inputting the state classification result of the second sample object in the each piece of second training data into a preset second deep learning model to obtain predicted data of the desired configuration information of the pulse generator (11);
updating a model parameter of the second deep learning model according to the predicted data of the desired configuration information of the pulse generator (11) and the annotated data of the desired configuration information of the pulse generator (11); and
detecting whether a preset second training end condition is satisfied; in response to satisfying the second training end condition, using the trained second deep learning model as the parameter configuration model; and in response to not satisfying the second training end condition, continuing training the second deep learning model by using next piece of second training data.

4. The pulse generator of claim 1, wherein the processor is configured to acquire the desired configuration information of the pulse generator (11) corresponding to the state classification result of the patient in the following manner:
acquiring a first correspondence relationship between the state classification result and the desired configuration information; and
determining the desired configuration information of the pulse generator (11) corresponding to the state classification result of the patient according to the state classification result of the patient and the first correspondence relationship.

5. The pulse generator of claim 3 or 4, wherein the desired configuration information of the pulse generator (11) is configured to indicate a parameter value of each of at least one of the following stimulation parameters:
a voltage amplitude of a stimulation pulse signal, a pulse width of the stimulation pulse signal, and a frequency of the stimulation pulse signal; or
identification information of an electrode contact for delivering electrical stimulation.

6. The pulse generator of claim 1, wherein an acquiring process of the first preset duration comprises:
inputting the state classification result of the patient and power of the pulse generator (11) into a duration calculation model to obtain the first preset duration,
wherein a training process of the duration calculation model comprises:
acquiring a third training set, wherein the third training set comprises a plurality of pieces of third training data, and each piece of third training data among the plurality of pieces of third training data comprises a state classification result of a third sample object, power of a sample pulse generator, and annotated data of the first preset duration; and
for the each piece of third training data in the third training set, performing the following processing:
inputting the state classification result of the third sample object in the each piece of third training data and the power of the sample pulse generator into a preset third deep learning model to obtain predicted data of the first preset duration;
updating a model parameter of the third deep learning model according to the predicted data of the first preset duration and the annotated data of the first preset duration; and
detecting whether a preset third training end condition is satisfied; in response to satisfying the third training end condition, using the trained third deep learning model as the duration calculation model; and in response to not satisfying the third training end condition, continuing training the third deep learning model by using next piece of third training data.

7. The pulse generator of claim 1, wherein an acquiring process of the first preset duration comprises:
acquiring a second correspondence relationship between power and the first preset duration; and determining the first preset duration corresponding to power of the pulse generator (11) at a current moment according to the power of the pulse generator (11) at the current moment and the second correspondence relationship.

8. The pulse generator of claim 1, wherein a disease of the patient comprises at least one of the following:
seizure disorder, depressive disorder, bipolar disorder, anxiety disorder, post-traumatic stress disorder, obsessive-compulsive disorder, behavioral disorder, mood disorder, memory disorder, mental state disorder, tremor, Parkinson's disease, Huntington's disease, Alzheimer's disease, addictive disorder, or autism.

9. A medical system, comprising:
the pulse generator (11) of any one of claims 1 to 8;
the electrode wire (12) configured to sense the electrophysiological activity of the patient and deliver electrical stimulation; and
a program control device (20) configured to establish a program control connection with the pulse generator (11), receive a range configuration operation, and determine an adjustable numerical range corresponding to each stimulation parameter of the at least one stimulation parameter of the pulse generator (11) in response to the range configuration operation,
wherein the pulse generator (11) is configured to perform adaptive adjustment within the adjustable numerical range corresponding to each stimulation parameter.

10. A computer-readable storage medium storing a computer program which, when executed by a processor, causes the processor to fulfill the functions of the pulse generator of any one of claims 1 to 8.
